# EUROPEAN PATENT APPLICATION

(11) **EP 2 933 327 A1**
(43) Date of publication of application: **21.10.2015**
(21) Application number: 13862002.6
(22) Date of filing: 11.12.2013
(51) Int. Cl.: C12M 1/32, C12M 1/00, C12Q 1/04

(54) **CANCER CELL ISOLATION DEVICE AND CANCER CELL ISOLATION METHOD**

(30) Priority: 12.12.2012 JP 2012271480
(71) Applicant: Hitachi Chemical Co., Ltd., Chiyoda-ku Tokyo 100-6606 (JP)
(72) Inventor: KANBARA, Hisashige, Chikusei-shi Ibaraki 308-0861 (JP); SUZUKI, Takahiro, Chikusei-shi Ibaraki 308-0861 (JP); KIKUHARA, Yoshihito, Chikusei-shi Ibaraki 308-0861 (JP); KANETOMO, Masafumi, Tokyo 168-0082 (JP)
(74) Representative: Hoffmann Eitle
(86) International application number: PCT/JP2013/083232
(87) International publication number: WO 2014/092127

(57) **Abstract**

Disclosed is to isolate cancer cells trapped on a filter without any damage.

In a cancer cell isolation device (1), cancer cells X trapped on a filter (100) are captured by a camera (30) as an image capturing unit, and the cancer cells X are carried one by one by using a handling unit (40) while the filter (100) and a storage container (200) are moved by an X-axis movement stand (14), a Y-axis movement stand (16), and a Z-axis movement stand (18) as movement units while the captured image is output from an output unit (32). Accordingly, it is possible to isolate the cancer cells on the filter without damaging the cancer cells and hence to appropriately perform an observation or the like.

## Description

### Technical Field

The present invention relates to a cancer cell isolation device and a cancer cell isolation method.

### Background Art

A cancer is ranked at a high position of the cause of death in all countries of the world. In Japan, 300,000 people or more die every year due to the cancer. Thus, there has been a desire to early discover and treat the cancer. The death of the people due to the cancer is mostly caused by the metastasis and recurrence of the cancer. The metastasis and recurrence of the cancer occurs when the cancer cell is fixed and infiltrated into blood vessel walls of other organ tissues while moving from an origin through blood vessels or lymphatic vessels so as to form minute metastases therein. The cancer cell which is circulated inside a person's body through the blood vessels or the lymphatic vessels is called a circulating tumor cell (hereinafter, referred to as a "CTC").

If the presence and the amount of the cancer cell (CTC) in the blood vessels that has a possibility of causing the metastasis and recurrence of the cancer may be measured, the cancer may be treated due to the measurement. As a related art of trapping the cancer cell in blood, for example, a configuration disclosed in Patent Literature 1 is known. In the related art disclosed in Patent Literature 1, the cancer cell in blood is trapped by a filter. Here, a method of manufacturing a filter using a semiconductor technique, a shape of a cell unit storing a filter, and a passage for blood and treatment solution are disclosed.

### Citation List

### Patent Literature

Patent Literature 1: US Patent No. 2011/0053152

### Summary of Invention

### Technical Problem

In recent years, there has been a demand to observe each cancer cell in order to more accurately recognize the state of the cancer cells in blood vessels. However, for example, even when the cancer cells are trapped on the filter by using the technique disclosed in Patent Literature 1, each cancer cell is not easily observed. Further, since the cancer cells on the filter are easily and elastically deformed, the treatment thereof is difficult. In addition, when the cancer cells are damaged, the cancer cells may not be appropriately observed.

The invention is made in view of the above-described circumstance, and an object thereof is to provide a cancer cell isolation device and a cancer cell isolation method capable of isolating cancer cells trapped on a filter without damaging the cancer cells.

### Solution to Problem

In order to achieve the object, according to an aspect of the invention, provided is a cancer cell isolation device which stores cancer cells trapped on a filter having a plurality of penetration holes in a storage container in an isolated state, the cancer cell isolation device including: an image capturing unit which captures the filter trapping the cancer cells in a magnified state; a movement unit which moves the filter and the storage container; and a handling unit which carries the cancer cells one by one while moving the filter and the storage container by the movement unit based on the image captured by the image capturing unit.

Further, according to another aspect of the invention, provided is a cancer cell isolation method of storing cancer cells trapped on a filter having a plurality of penetration holes in a storage container in an isolated state, the cancer cell isolation method including: capturing the filter trapping the cancer cells in a magnified state; and carrying the cancer cells one by one while moving the filter and the storage container based on the image captured in the capturing of the filter.

According to the cancer cell isolation device and the cancer cell isolation method, the cancer cells trapped on the filter are captured by the image capturing unit, and the cancer cells are carried one by one by the handling unit while the filter and the storage container are moved by the movement unit based on the captured image. Accordingly, it is possible to isolate the cancer cells on the filter without damaging the cancer cells and hence to appropriately perform an observation or the like.

Here, as an effective configuration of the above-described effects, specifically, the handling unit includes a suction nozzle, and the cancer cell is carried while the cancer cell is adsorbed to the front end of the suction nozzle.

Further, as another effective configuration of the above-described effects, specifically, the handling unit includes a micro tweezers (micro pincette), and the cancer cell is carried while the cancer cell is gripped by the micro tweezers.

Further, the cancer cell isolation device further includes a position recognition unit which acquires position information for specifying the position of the cancer cell on the filter based on the image of the filter captured by the image capturing unit, wherein the cancer cell on the filter is carried by the handling unit based on the position information of the cancer cell acquired by the position recognition unit.

As described above, since the position information of the cancer cell captured by the image capturing unit is acquired by the position recognition unit and the cancer cell is carried based on the position information, it is possible to more accurately recognize the position information of the cancer cell and to efficiently carry the cancer cell.

Further, a reference mark representing the arrangement of the penetration hole is provided on the filter. Furthermore, the filter is moved by the movement unit and the position information of the cancer cell is acquired by the position recognition unit based on the reference mark.

As described above, since the position information of the cancer cell is acquired by using the reference mark, it is possible to more accurately recognize the position of the cancer cell. Further, since the filter is moved by using the reference mark, it is possible to more accurately position and move the filter and hence to more efficiently isolate the cancer cells.

### Advantageous Effects of Invention

According to the invention, it is possible to provide a cancer cell isolation device and a cancer cell isolation method capable of isolating cancer cells trapped on a filter without damaging the cancer cells.

### Brief Description of Drawings

Fig. 1 is a configuration diagram illustrating a cancer cell isolation device according to an embodiment.
Fig. 2 is a diagram illustrating a configuration of a filter according to an embodiment.
Fig. 3 is a diagram illustrating a configuration of a filter unit according to an embodiment.
Fig. 4 is a configuration diagram illustrating a cancer cell isolation device according to a modified example.
Fig. 5 is a configuration diagram illustrating a cancer cell isolation device according to a modified example.
Fig. 6 is a diagram illustrating a configuration of a filter according to a modified example.
Fig. 7 is a diagram illustrating a configuration of a filter according to a modified example.
Fig. 8 is a diagram illustrating a configuration of a filter unit according to a modified example.
Fig. 9 is a diagram illustrating a configuration of a filter unit according to a modified example.

### Description of Embodiments

Hereinafter, an embodiment of the invention will be described in detail with reference to the accompanying drawings. In addition, the same reference sign will be given to the same component in the description of the drawings, and the repetitive description thereof will not be presented.

### (Cancer Cell Isolation Device)

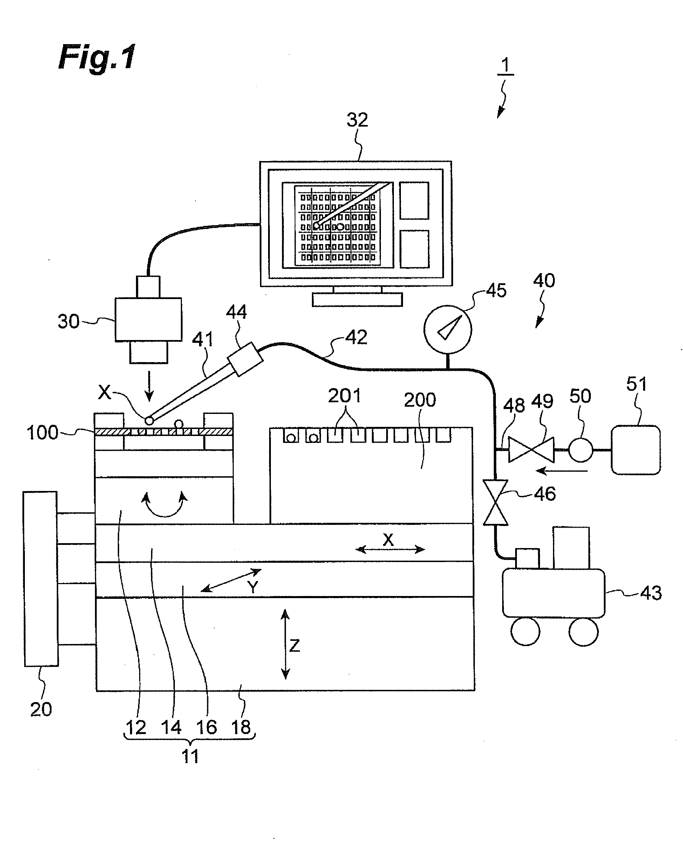

Fig. 1 illustrates a configuration of a cancer cell isolation device according to an embodiment of the invention. A cancer cell isolation device 1 is a device which moves a cancer cell X on a filter 100 trapping cancer cells one by one to a plurality of storage portions 201 provided in a storage container 200. The cancer cell isolation device 1 includes a movement stand 11 which moves the filter 100, a movement stand control unit 20 which controls the operation of the movement stand 11, a camera 30 which observes the filter 100 in a magnified state, an output unit 32 which outputs an image captured by the camera 30, and a handling unit 40 which moves the cancer cell X from the filter 100 to the storage container 200.

The movement stand 11 includes a rotation stand 12 which rotates the filter 100 along the horizontal plane, an X-axis movement stand 14 and a Y-axis movement stand 16 which move the filter 100 and the storage container 200 along the horizontal plane in two directions perpendicular to each other, and a Z-axis movement stand 18 which moves the filter 100 and the storage container 200 in the up and down direction. When these components are operated based on an instruction from the movement stand control unit 20, the filter 100 and the storage container 200 are moved so that the cancer cell X is easily manipulated by the handling unit 40.

The camera 30 is attached so as to capture a principal surface of the filter 100 in a magnified state from the upside. Then, when an image which is captured by the camera 30 is output from the output unit 32 connected to the camera 30, an operator who operates the cancer cell isolation device 1 may check the image.

The handling unit 40 includes a handling nozzle 41 (a suction nozzle), a negative pressure pump 43 which is connected to the handling nozzle 41 through a tube 42, and a nozzle movement stand 44 which moves the handling nozzle 41. A pressure gauge 45 is attached to a halfway position of the tube 42, and is used to monitor a gas pressure inside the tube 42. Further, a valve 46 is attached to the vicinity of the end of the tube 42 near the negative pressure pump 43. Further, the tube 42 is branched into two parts on the side of the handling nozzle 41 in relation to the valve 46. A valve 49, a regulator 50, and a compressed air tank 51 are attached to a tube 48 connected to the tube 42 in this order from the dividing point. That is, when compressed air inside the compressed air tank 51 is output while being adjusted in pressure by the regulator 50 at the time point in which the valve 46 is closed and the valve 49 is opened, the compressed air may flow through the tube 42.

In this way, the handling unit 40 has the configuration. When the negative pressure pump 43 is driven while the valve 46 is opened and the valve 49 is closed, the air inside the tube 42 is suctioned by the negative pressure pump 43, and hence the cancer cell X may be absorbed to the front end of the handling nozzle 41. When the cancer cell X is attached to the front end of the handling nozzle 41, the pressure inside the tube 42 becomes a negative pressure. Accordingly, it is possible to check a state where the cancer cell X is attached to the front end of the handling nozzle 41 by checking the numerical value of the pressure gauge 45. Further, the handling nozzle 41 which absorbs the cancer cell X is made to approach a place (for example, the storage portion 201 of the storage container 200) where the cancer cell X desired to be placed by the linkage operation between the nozzle movement stand 44 and the movement stand 11, and the compressed air is output from the compressed air tank 51 while the valve 46 is closed and the valve 49 is opened so that the compressed air is charged into the tube 42. Accordingly, the cancer cell X attached to the handling nozzle 41 may be separated from the handling nozzle 41.

Although the configuration of the filter 100 will be described in detail later, the filter is a plate-shaped member provided with a plurality of penetration holes in the thickness direction of the principal surface. When blood as a test liquid passes through the penetration holes of the filter 100 by using a filter unit to be described later, the filter trapping the cancer cell by using the hole diameter of the penetration hole is attached to the cancer cell isolation device. Further, the storage container 200 is a container which stores the isolated cancer cells X one by one while one cancer cell is input and stored into the defined storage portion 201.

### (Cancer Cell Isolation Method)

Here, a cancer cell isolation method using the cancer cell isolation device 1 according to the embodiment will be described. First, the filter 100 is attached to the cancer cell isolation device 1. At this time, the cancer cell X is already attached to the filter 100, and a cleaning/dying treatment or the like is performed if necessary. In a case where the filter 100 is attached to the cancer cell isolation device 1, the rotation stand 12 is used to align the movement directions of the X-axis movement stand 14 and the Y-axis movement stand 16 to the alignment directions of the penetration holes of the filter. In addition, the movement directions of the X-axis movement stand 14 and the Y-axis movement stand 16 are perpendicular to each other. Here, a case will be described in which the penetration holes are arranged along the perpendicular lines of the filter 100.

Next, the camera 30 is brought into focus with respect to the principal surface of the filter 100, that is, the attachment surface of the cancer cell X, and the filter is moved by the X-axis movement stand 14, the Y-axis movement stand 16, and the Z-axis movement stand 18 so that the attachment position of the cancer cell X in the principal surface of the filter 100 is captured by the camera. Then, the camera 30 is brought into focus with respect to the cancer cell X. At this time, it is desirable that the magnification of the camera 30 be set to a magnification in which both the principal surface of the filter 100 and the cancer cell X become focused.

Next, the front end of the handling nozzle 41 of the handling unit 40 is moved to the vicinity of the cancer cell X by using the nozzle movement stand 44. At this time, the distance between the front end of the handling nozzle 41 and the cancer cell X is set to about 10 µm.

Subsequently, the filter 100 is moved by the X-axis movement stand 14, the Y-axis movement stand 16, and the Z-axis movement stand 18 so that the front end of the handling nozzle 41 contacts the cancer cell X. Further, the negative pressure pump 43 is driven so that the cancer cell X is adsorbed to the front end of the handling nozzle 41 by the suction thereof. Here, it is possible to check a state where the cancer cell X is adsorbed based on the suction pressure measured by the pressure gauge 45.

Next, the Z-axis movement stand 18 is driven so that the filter 100 is moved downward by several mm (so as to be separated from the handling nozzle 41), the filter 100 disposed below the handling nozzle 8 is moved by using the X-axis movement stand 14 and the Y-axis movement stand 16, and the storage container 200 is moved so that a predetermined storage portion 201 of the storage container 200 is disposed instead.

Next, the storage container 200 is moved upward by the Z-axis movement stand 18 so that the storage portion 201 approaches the cancer cell X of the front end of the handling nozzle 41. In an approached state, the pressure inside the tube 42 is switched from a negative pressure to a pressurized pressure in a manner such that the valves 46 and 49 are opened or closed and compressed air is supplied from the compressed air tank 51. Then, the cancer cell X is moved from the front end of the handling nozzle 41 to the storage portion 201 by the compressed air. Accordingly, one cancer cell X on the filter 100 is separately stored in the storage portion 201 of the storage container 200.

Subsequently, the storage container 200 and the filter 100 are switched by the driving of the X-axis movement stand 14, the Y-axis movement stand 16, and the Z-axis movement stand 18 so that the filter 100 is moved to the lower portion of the camera 30. Then, the operation is repeated so as to isolate the cancer cell X on the filter 100.

### (Filter)

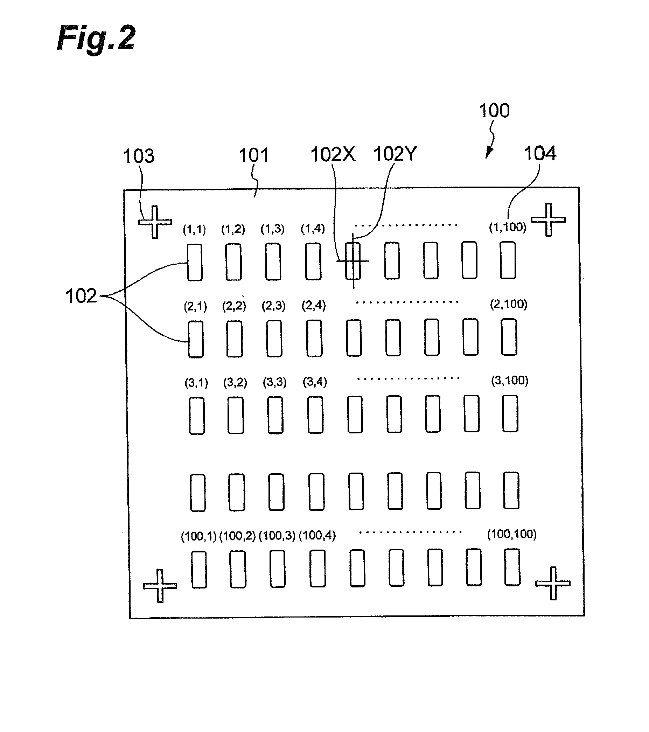

Here, the filter 100 which is used to isolate the cancer cell will be described with reference to Fig. 2. Fig. 2 is a diagram illustrating a configuration of the filter 100. The filter 100 shown in Fig. 2 has a structure in which a plurality of rectangular penetration holes 102 is formed in the thickness direction of the principal surface of the sheet 101. In the filter 100 of Fig. 2, a plurality of penetration holes 102 is disposed in a matrix shape. Further, an alignment mark 103 used for the alignment is disposed at four corners of the sheet 101. Further, an individual identification character 104 (a reference mark) is marked on the sheet 101 in the vicinity of the penetration hole so as to correspond to each penetration hole 102.

The alignment mark 103 is used to adjust the alignment of the penetration holes 102, that is, the direction of the sheet 101 and the movement directions of the X-axis movement stand 14 and the Y-axis movement stand 16 by using the rotation stand 12 when the filter 100 is disposed on the cancer cell isolation device 1. That is, the alignment mark 103 which is disposed at four corners of the filter 100 is used so as to correspond to the positional relation (the arrangement and the rectangular shape of the penetration hole 102) of the penetration hole 102 of the filter 100. Specifically, for example, when the alignment mark 103 has a "cross" shape as shown in Fig. 2, the direction indicated by the cross mark matches the short axis direction 102X and the long axis direction 102Y of the penetration hole 102. Accordingly, when the filter is fixed to the cancer cell isolation device 1 while the cross-shaped alignment mark 103 is correlated with the movement directions of the X-axis movement stand 14 and the Y-axis movement stand 16, the movement directions of the X-axis movement stand 14 and the Y-axis movement stand 16 match the long axis direction and the short axis direction of the penetration hole 102. For this reason, it is possible to easily perform more accurate movement of the handling nozzle 41 and the filter 100.

Further, the identification character 104 which is marked in the vicinity of the penetration hole 102 is marked so that two numerals are parenthesized. Here, the first numeral of two numerals indicates the row of the penetration hole 102, and the second numeral indicates the column of the penetration hole 102. By using the identification character 104, the penetration hole 102 of the top surface of the filter 100 may be specified. In this way, since the position of the penetration hole 102 is specified by using the identification character 104, it is possible to easily specify the penetration hole 102 of the filter 100 trapping the cancer cell X by using the identification character 104 of the penetration hole 102 and hence to efficiently perform the isolation operation. In addition, the information for specifying the position of the penetration hole 102 is not limited to the arrangement of two numerals. For example, a symbol or an alphabet may be freely used. Further, there is no need to carve a character for all penetration holes 102 on the filter 100, and only a character capable of distinguishing each penetration hole 102 may be provided. Further, instead of the method of identifying each penetration hole 102, for example, a configuration may be employed in which nine penetration holes 102 of 3 rows by 3 columns are grouped as one block and each block is identified.

### (Filter Unit)

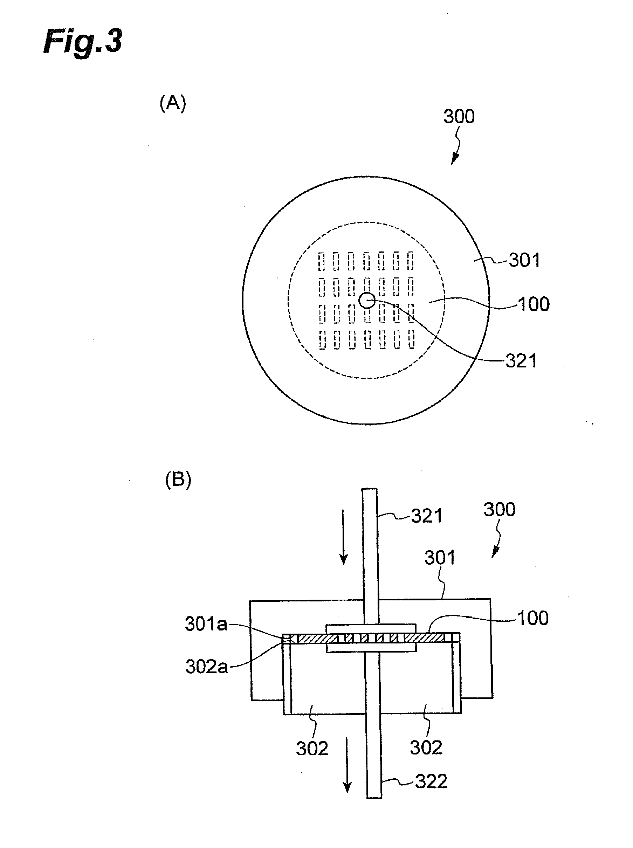

Next, a filter unit used to capture the cancer cell in the blood vessel using the filter 100 will be described with reference to Fig. 3. Fig. 3 is a diagram illustrating a configuration of a filter unit 300, where Fig. 3(A) is a top view illustrating the filter unit 300 and Fig. 3(B) is a schematic cross-sectional view thereof. In Fig. 3, the filter unit 300 having a circular external shape and used to capture the cancer cell using the filter 100 having a circular sheet will be described. The filter unit 300 is formed by the combination of an upper nut 301 having a concave cross-sectional shape and a lower nut 302 having a columnar shape. The filter 100 is fixed so as to be nipped between a top surface 302a of the lower nut 302 and a bottom surface 301a of a concave portion of the upper nut 301. The bottom surface 301a and the top surface 302a are respectively provided with void portions 311 and 312 provided at the positions respectively corresponding to the penetration holes 102 of the filter 100. Further, the upper nut 301 is provided with an upper nut nozzle 321 which communicates with the void portion 311 so as to supply blood from the outside. Similarly, the lower nut 302 is also provided with a lower nut nozzle 322 which communicates with the void portion 312 so as to supply blood from the outside.

In the filter unit 300 having the configuration, the filter 100 is fixed while being nipped between the upper nut 301 and the lower nut 302, and a test liquid such as blood is supplied from the end of the upper nut nozzle 321. The test liquid which reaches the void portion 311 from the upper nut nozzle 321 passes through the penetration hole 102 of the filter 100, and is discharged from the void portion 312 to the outside through the lower nut nozzle 322. At this time, the cancer cell having a diameter larger than the penetration hole 102 is trapped by the penetration hole 102 of the filter 100. Subsequently, a treatment liquid such as a cleaning liquid and a dying liquid for observing the cancer cell is sequentially supplied from the upper nut nozzle 321 so as to clean and dye the cancer cell on the filter 100. After these treatments, the filter unit 300 may be extracted by the separation of the upper nut 301 and the lower nut 302 in accordance with the operation of loosening the upper nut 301 and the lower nut 302. The extracted filter unit is attached to the cancer cell isolation device 1, and the cancer cell is isolated while being visually recognized. In addition, the screw loosening operation may be performed while the lower nut 302 is fixed to a different fixing stand (not shown) so that any vibration is not applied to the filter 100.

According to the cancer cell isolation device and the cancer cell isolation method, the cancer cells X trapped on the filter 100 are captured by the camera 30 as an image capturing unit, and the cancer cells X are carried one by one by using the handling unit 40 while the filter 100 and the storage container 200 are moved by the X-axis movement stand 14, the Y-axis movement stand 16, and the Z-axis movement stand 18 as the movement units based on the captured image is output from the output unit 32. Accordingly, it is possible to isolate the cancer cells on the filter without damaging the cancer cells and hence to appropriately perform an observation or the like.

### (Modified Example)

Hereinafter, a modified example according to the filter used in the cancer cell isolation device and the cancer cell isolation device will be described.

### (Modified Example of Cancer Cell Isolation Device)

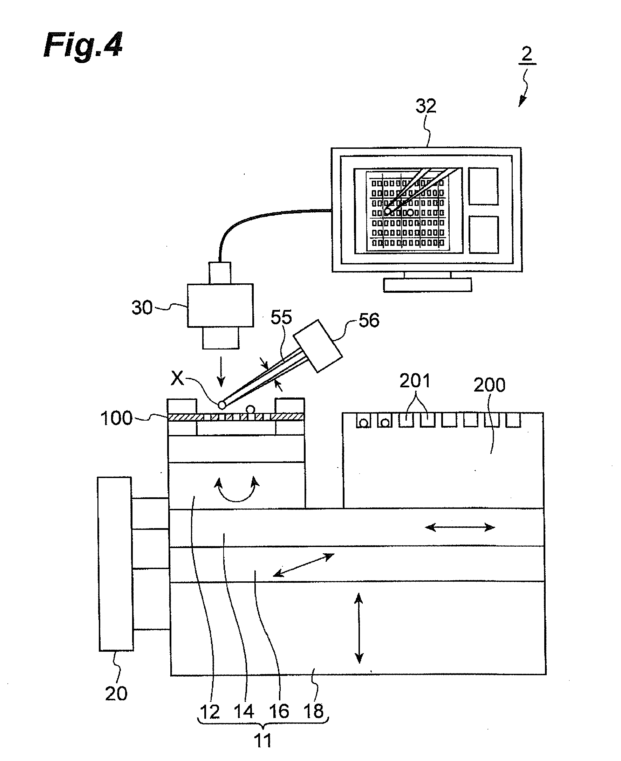

Fig. 4 is a diagram illustrating a first modified example of a cancer cell isolation device. A cancer cell isolation device 2 of Fig. 4 and the cancer cell isolation device 1 of Fig. 1 are different from each other as below. That is, a micro tweezers 55 (micro pincette) is used as a handling unit in the cancer cell isolation device 2 instead of the handling unit 40. The micro tweezers 55 is attached to a tweezers movement stand 56, and the front end thereof is movable in the image capturing region of the camera 30. In this way, even when the micro tweezers 55 is used as the handling unit, the cancer cell X may be carried to the storage portion 201 of the storage container 200 similarly to the case of using the handling nozzle 41 in a manner such that the camera 30 is brought into focus with respect to the cancer cell X of the principal surface of the filter 100, the front end of the micro tweezers 55 is moved to the vicinity of the cancer cell X, the cancer cell X is gripped by the front end of the micro tweezers 55, and the filter 100 and the storage container 200 are moved.

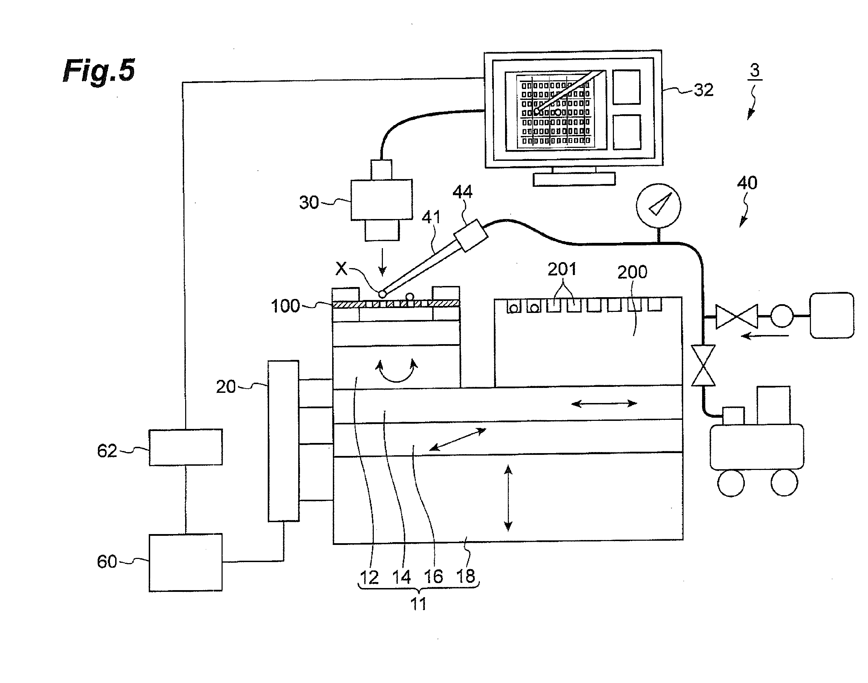

Next, a second modified example of a cancer cell isolation device will be described with reference to Fig. 5. A cancer cell isolation device 3 of Fig. 5 and the cancer cell isolation device 1 of Fig. 1 are different from each other as below. That is, the information representing the position of the cancer cell X on the filter 100 is acquired by using the image captured by the camera 30. The movement stand control unit 20 moves the rotation stand 12, the X-axis movement stand 14, the Y-axis movement stand 16, and the Z-axis movement stand 18 based on this information. In the cancer cell isolation device 3 of Fig. 5, the image captured by the camera 30 is transmitted to a recognition unit 62 (a position recognition unit) connected to the output unit 32. Then, the recognition unit 62 specifies the position of the penetration hole 102 of the filter 100 where the cancer cell X is trapped. That is, the recognition unit 62 acquires the position information for specifying the position of the cancer cell X. As the position information used herein, the identification character 104 given to each penetration hole 102 of the filter 100 may be exemplified. Then, the information involved with the identification character 104 specifying the penetration hole 102 having the cancer cell X trapped thereto is acquired as the position information of the cancer cell X by the recognition unit 62. Next, the position information of the cancer cell X is transmitted from the recognition unit 62 to the control unit 60.

Next, the control unit 60 generates an instruction involved with the movement of the filter 100 and the storage container 200 carrying the cancer cell X based on the position information of the cancer cell X. Specifically, the control unit generates an instruction involved with the movement of the movement stand 11 moving the cancer cell X located at a specific position of the filter 100 specified by the recognition unit 62 with respect to the storage portion 201 of the storage container 200. Further, the control unit 60 may generate an instruction involved with the driving of the handling nozzle 41 for the handling unit 40. Then, when the movement stand control unit 20 is driven and the handling nozzle 41 is operated in accordance with the instruction from the control unit 60, the cancer cell X on the filter 100 may be moved from the filter 100 to the storage container 200.

In addition, in the cancer cell isolation device 3, the operation of isolating the cancer cell and the operation of acquiring the position information involved with the position of the cancer cell X on the filter 100 are performed while the filter 100 is fixed to the cancer cell isolation device 3. However, the filter 100 may be separated once after the position information involved with the position of the cancer cell X on the filter 100 is acquired. Further, the operation of isolating the cancer cell and the operation of acquiring the position information involved with the position of the cancer cell X on the filter 100 may be performed by different devices. In this way, even when the filter 100 is separated from the cancer cell isolation device once, it is possible to promptly perform the isolation operation by accurately attaching the filter 100 to the device during the cancer cell isolating operation in a manner such that the position information of the cancer cell X on the filter 100 is stored in the cancer cell isolation device 3. In order to accurately attach the filter 100 to the cancer cell isolation device 3, the alignment mark 103 (see Fig. 2) provided in the filter 100 is usefully adopted.

### (Modified Example of Filter)

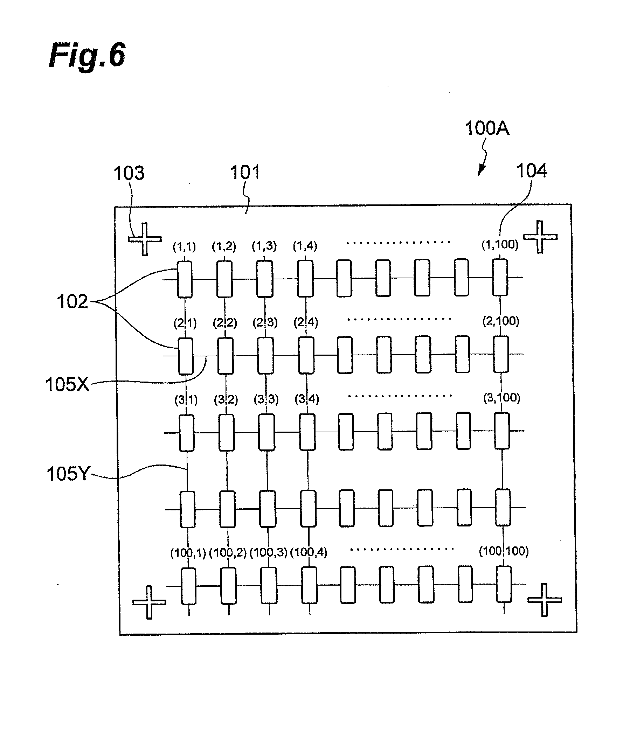

Next, a modified example of a filter will be described with reference to Fig. 6. A filter 100A of Fig. 6 and the filter 100 of Fig. 2 are different from each other as below. That is, lines 105X and 105Y extending in two perpendicular directions are provided so as to connect the adjacent penetration holes 102. The line 105X is a line which extends in the short axis direction of the penetration hole 102 and connects the middle points of the adjacent penetration holes 102. Further, the line 105Y is a line which extends in the long axis direction of the penetration hole 102 and connects the middle points of the adjacent penetration holes 102. In this way, since the lines 105X and 105Y connecting the penetration holes 102 are marked on the sheet 101 in the filter 100A, it is possible to more accurately position the filter 100 by the positioning operation of the X-axis movement stand 14 and the Y-axis movement stand 16 with reference to the lines 105X and 105Y as well as the alignment mark 103 provided at four corners of the sheet 101 and to more appropriately move the filter 100 in response to the position of the penetration hole 102 trapping the cancer cell X.

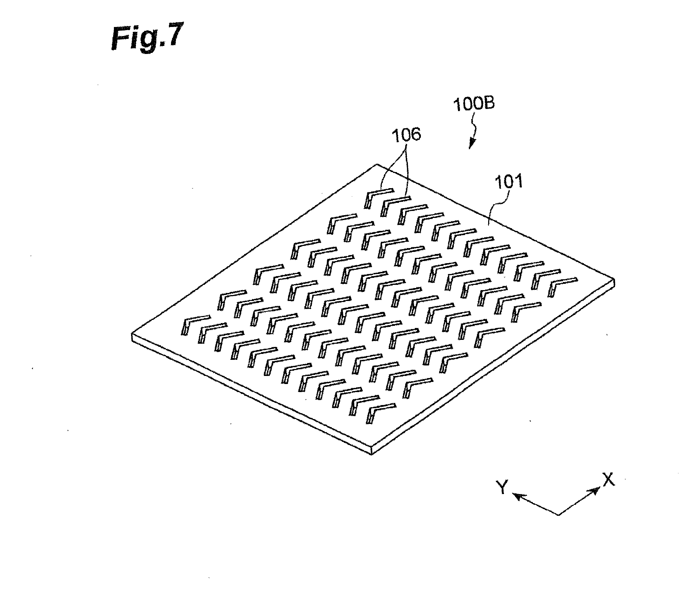

Next, an example of a case where the shape of the penetration hole of the filter is changed will be described with reference to Fig. 7. In a filter 100B shown in Fig. 7, the shape of the penetration hole 106 of the top surface of the sheet 101 is formed as a wave shape. The penetration hole having a wave shape is formed so that the ends of the rectangular or round rectangular punched holes of the top surface of the sheet 101 are connected to each other while forming a predetermined intersection angle therebetween. In the filter 100B of Fig. 7, the wave shape of the penetration hole 106 is formed in the X direction. In this way, the shape of the penetration hole of the filter may be appropriately changed.

### (Modified Example of Filter Unit)

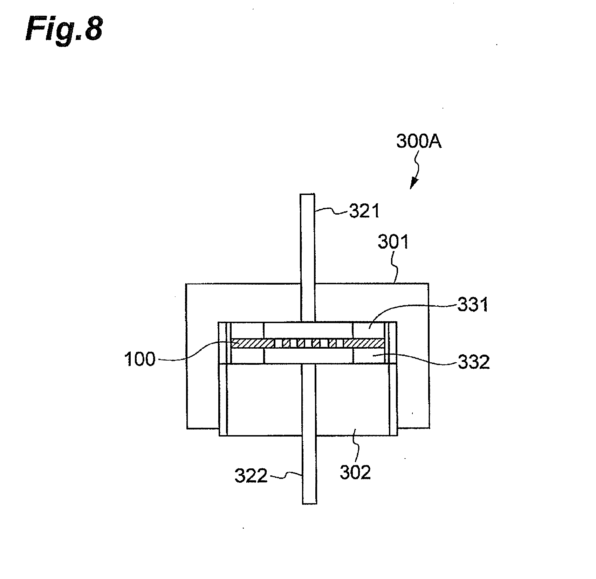

Next, a modified example of a filter unit will be described with reference to Fig. 8. Fig. 8 is a cross-sectional view illustrating the modified example of the filter unit and corresponds to Fig. 3(B). A filter unit 300A of Fig. 8 and the filter unit 300 of Fig. 3 are different from each other as below. That is, the upper and lower peripheral edges of the filter 100 are fixed between the upper nut 301 and the lower nut 302 while being nipped between an upper frame plate 331 and a lower frame plate 332. In the filter unit 300A of Fig. 8, the upper frame plate 331 and the lower frame plate 332 are respectively bonded to the principal surface (the upper surface and the lower surface) of the filter 100 so as to interpose the filter 100 therebetween. That is, the filter 100, the upper frame plate 331, and the lower frame plate 332 are integrated with one another. It is desirable that each of the upper frame plate 331 and the lower frame plate 332 be a member that does not form a gap when the filter 100 is attached between the upper nut 301 and the lower nut 302. For example, silicone rubber or the like may be used.

Here, since the upper frame plate 331 and the lower frame plate 332 fix the filter 100 while the filter 100 is not loosened, the deformation of the filter 100 may be prevented. Further, in a case where the filter 100 is extracted from the filter unit 300A, the filter is extracted while the upper frame plate 331 and the lower frame plate 332 are attached thereto. For this reason, since it is possible to prevent the top surface of the filter from being wrinkled even when the filter 100 is moved, it is possible to prevent the damage of the cancer cell X trapped on the filter 100 and to prevent degradation in the workability caused by the deformation of the filter 100 even when the filter 100 is attached to the cancer cell isolation device and the subsequent operations are performed.

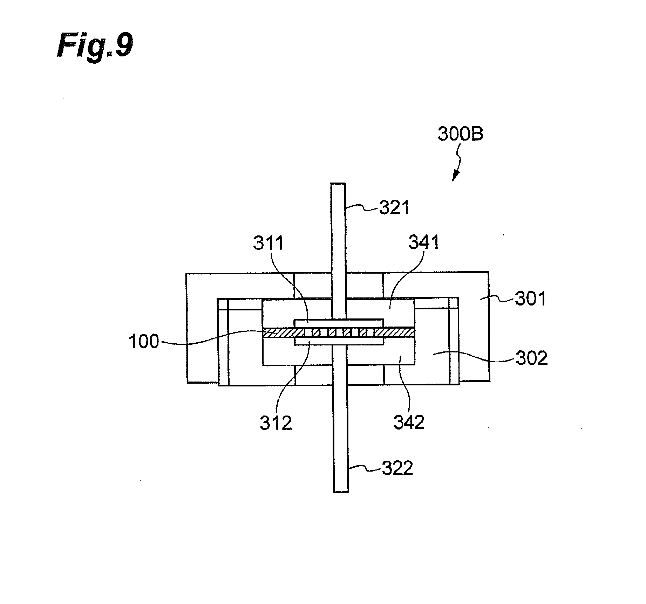

Next, another modified example of a filter unit will be described with reference to Fig. 9. The filter unit 300A of Fig. 8 and the filter unit 300 of Fig. 3 are different from each other as below. That is, the filter 100 is fixed between the upper nut 301 and the lower nut 302 while the filter 100 is nipped between the upper plate 341 and the lower plate 342 forming the void portions 311 and 312. The upper nut nozzle 321 connected to the void portion 311 is attached to the upper plate 341, and the lower nut nozzle 322 connected to the void portion 312 is attached to the lower plate 342. In such a configuration, the upper nut 301 and the lower nut 302 may be used many times since the nuts do not contact a test liquid. Thus, for example, metal having high durability may be used as the upper nut 301 and the lower nut 302. Meanwhile, for example, when resinous consumables are manufactured/used as the upper plate 341 and the lower plate 342 contacting a test liquid, it is possible to reduce the cost involved with the isolation of the cancer cell compared to the case where the filter unit 300 of Fig. 3 is used.

While the embodiment of the invention has been described, the invention is not limited to the embodiment, and may be modified into various forms.

### Reference Signs List

1, 2, 3 cancer cell isolation device, 11 movement stand, 20 movement stand control unit, 30 camera, 32 output unit, 40 handling unit, 41 handling nozzle, 55 micro tweezers, 100 filter, 102, 106 penetration hole, 200 storage container, 201 storage portion

## Claims

1. A cancer cell isolation device which stores cancer cells trapped on a filter having a plurality of penetration holes in a storage container in an isolated state, the cancer cell isolation device comprising:
an image capturing unit which captures the filter trapping the cancer cells in a magnified state;
a movement unit which moves the filter and the storage container; and
a handling unit which carries the cancer cells one by one while moving the filter and the storage container by the movement unit based on the image captured by the image capturing unit.

2. The cancer cell isolation device according to claim 1,
wherein the handling unit includes a suction nozzle, and
wherein the cancer cell is carried while the cancer cell is adsorbed to the front end of the suction nozzle.

3. The cancer cell isolation device according to claim 1,
wherein the handling unit includes a micro tweezers, and
wherein the cancer cell is carried while the cancer cell is gripped by the micro tweezers.

4. The cancer cell isolation device according to any one of claims 1 to 3, further comprising:
a position recognition unit which acquires position information for specifying the position of the cancer cell on the filter based on the image of the filter captured by the image capturing unit,
wherein the cancer cell on the filter is carried by the handling unit based on the position information of the cancer cell acquired by the position recognition unit.

5. The cancer cell isolation device according to claim 4,
wherein a reference mark representing the arrangement of the penetration hole is provided on the filter, and
wherein the filter is moved by the movement unit and the position information of the cancer cell is acquired by the position recognition unit based on the reference mark.

6. A cancer cell isolation method of storing cancer cells trapped on a filter having a plurality of penetration holes in a storage container in an isolated state, the cancer cell isolation method comprising:
capturing the filter trapping the cancer cells in a magnified state; and
carrying the cancer cells one by one while moving the filter and the storage container based on the image captured in the capturing of the filter.
